# EUROPEAN PATENT APPLICATION

(11) **EP 3 961 991 A1**
(43) Date of publication of application: **02.03.2022**
(21) Application number: 20197516.6
(22) Date of filing: 22.09.2020
(51) Int. Cl.: H04L 12/28, A61B 5/00, G16H 20/00

(54) **STORAGE DEVICE AND MEDICAL INSTRUMENT WITH SAME**

(30) Priority: 28.08.2020 TW 109129600
(71) Applicant: Key ASIC Inc., 302 Jubei City Hsinchu County (TW)
(72) Inventor: WU, Hao-Jen, 302 Jubei City (TW); LEE, Hsin-Kai, 114 Taipei City (TW)
(74) Representative: Zeitler Volpert Kandlbinder Patentanwälte Partnerschaft mbB

(57) **Abstract**

A storage device (1, 1a, 1b) is installed in a medical device (2, 2b). The storage device includes a storage module (11), a processing control module (12, 12a, 12b) and a wireless transmission module (13). A data generated by the medical device (2, 2b) is stored in the storage module (11). The processing control module (12, 12a, 12b) is electrically connected with the storage module (11). The wireless transmission module (13) is electrically connected with the processing control module (12, 12a, 12b). Under control of the processing control module (12, 12a, 12b), the data is acquired from the storage module (11) through the wireless transmission module (13) and transmitted to an external device (3).

## Description

### FIELD OF THE INVENTION

The present invention relates to a storage device, and more particularly to a storage device for a medical instrument.

### BACKGROUND OF THE INVENTION

Recently, due to the formation of various new viruses, pandemics have been triggered around the world, and caused a substantial increase in the number of severely-ill hospitalized patients. Consequently, the functionality of related medical instrument in use has played a very important role. Conventionally, when the use of the medical instrument generated the medical data about the patient's physical condition, the medical practitioners had to personally watch the medical data in front of the medical instrument and obtain the information about the disease condition of the patient through interpretation of the medical data. If these medical data need to be backed up or need to be moved to another device, the medical practitioners have to store the medical data to an external storage device before the medical data are transferred and stored to another device.

However, the use of the conventional medical instrument is time-consuming and laborious for the medical practitioners. The use of the conventional medical instrument is unable to cope with the sudden increase in patients. In addition, considerable psychological burden on medical practitioners is generated.

Therefore, it is important to increase the use convenience of the medical instrument in order to overcome the drawbacks of the conventional technologies.

### SUMMARY OF THE INVENTION

The present invention provides a storage device. The storage device is installed in a medical device. The data generated by the medical device can be transmitted to an external device through a wireless transmission module of the storage device.

The present invention also provides a medical instrument. The medical instrument includes a medical device and a storage device. The data generated by the medical device can be transmitted to an external device through a wireless transmission module of the storage device.

The other objects and advantages of the present invention will be understood from the disclosed technical features.

In accordance with an aspect of the present invention, a storage device is installed in a medical device. The storage device includes a storage module, a processing control module and a wireless transmission module. A data generated by the medical device is stored in the storage module. The processing control module is electrically connected with the storage module. The wireless transmission module is electrically connected with the processing control module. Under control of the processing control module, the data is acquired from the storage module and transmitted to an external device through the wireless transmission module.

In an embodiment, the storage device further includes an I/O interface module, and the I/O interface module is electrically connected with the medical device and the processing control module. Under control of the processing control module, the data from the medical device is transmitted and stored to the storage module through the I/O interface module.

In an embodiment, the processing control module includes a control unit and a dynamic switching unit. The dynamic switching unit is electrically connected between the control unit, the storage module, the I/O interface module and the wireless transmission module. The dynamic switching unit includes a register. When the wireless transmission module issues a first access signal to the dynamic switching unit, the first access signal is temporarily stored in the register. When the dynamic switching unit detects that the I/O interface module is in an idle state, the wireless transmission module is connected with the storage module through the dynamic switching unit under the control of the control unit, and the first access signal is transmitted to the storage module through the dynamic switching unit under the control of the control unit. In response to the first access signal, the data is acquired from the storage module through the wireless transmission module.

In an embodiment, when the I/O interface module issues a second access signal to the dynamic switching unit, the second access signal is temporarily stored in the register. When the dynamic switching unit detects that the wireless transmission module is in an interrupted state, the I/O interface module is connected with the storage module through the dynamic switching unit under the control of the control unit, and the second access signal is transmitted to the storage module through the dynamic switching unit under the control of the control unit. In response to the second access signal, the data generated by the medical device is stored into the storage module through the I/O interface module.

In an embodiment, the control unit is a central processing unit or a microcontroller.

In an embodiment, the processing control module includes a control unit and a dynamic switching unit. The control unit is electrically connected with the wireless transmission module, the dynamic switching unit is electrically connected between the control unit and the storage module, the dynamic switching unit includes a register, and the medical device includes a first access controller and a second access controller. Under control of the control unit, the first access controller or the second access controller is selectively connected with the storage module through the dynamic switching unit. When the second access controller issues a third access signal, the third access signal is temporarily stored in the register. When the dynamic switching unit detects that the first access controller is in an idle state, the second access controller is connected with the storage module through the dynamic switching unit under the control of the control unit, and the third access signal is transmitted to the storage module through the dynamic switching unit under the control of the control unit. In response to the third access signal, the medical device performs a data accessing operation on the storage module through the second access controller.

In an embodiment, when the first access controller issues a fourth access signal, the fourth access signal is temporarily stored in the register. When the dynamic switching unit detects that the second access controller is in an interrupted state, the first access controller is connected with the storage module through the dynamic switching unit under the control of the control unit, and the fourth access signal is transmitted to the storage module through the dynamic switching unit under the control of the control unit. In response to the fourth access signal, the medical device performs a data accessing operation on the storage module through the first access controller.

In an embodiment, the external device includes a man-machine operation interface. The data generated by the medical device is displayed on the man-machine operation interface, or the medical device is controlled according to a control command that is inputted through the man-machine operation interface.

In an embodiment, the processing control module is a central processing unit or a microcontroller.

In an embodiment, the external device is a smart phone, a tablet computer, a notebook computer or a personal computer.

In an embodiment, the wireless transmission module is a worldwide interoperability for microwave access (Wimax) module, a Wi-Fi module, a radio frequency (RF) module, a global positioning system (GPS) module or a global system for mobile communications (GSM) module.

In an embodiment, the medical device is an ultrasonic scanner, a ventilator, a fundus camera, a continuous positive airway pressure machine or a LED spectrum analyzer.

In accordance with another aspect of the present invention, a medical instrument is provided. The medical instrument includes a medical device and a storage device. The storage device is electrically connected with the medical device. The storage device includes a storage module, a processing control module and a wireless transmission module. A data generated by the medical device is stored in the storage module. The processing control module is electrically connected with the storage module. The wireless transmission module is electrically connected with the processing control module. Under control of the processing control module, the data is acquired from the storage module and transmitted to an external device through the wireless transmission module.

In an embodiment, the storage device further includes an I/O interface module, and the I/O interface module is electrically connected with the medical device and the processing control module. Under control of the processing control module, the data from the medical device is transmitted and stored to the storage module through the I/O interface module.

In an embodiment, the processing control module includes a control unit and a dynamic switching unit. The dynamic switching unit is electrically connected between the control unit, the storage module, the I/O interface module and the wireless transmission module. The dynamic switching unit includes a register. When the wireless transmission module issues a first access signal to the dynamic switching unit, the first access signal is temporarily stored in the register. When the dynamic switching unit detects that the I/O interface module is in an idle state, the wireless transmission module is connected with the storage module through the dynamic switching unit under the control of the control unit, and the first access signal is transmitted to the storage module through the dynamic switching unit under the control of the control unit. In response to the first access signal, the data is acquired from the storage module through the wireless transmission module.

In an embodiment, when the I/O interface module issues a second access signal to the dynamic switching unit, the second access signal is temporarily stored in the register. When the dynamic switching unit detects that the wireless transmission module is in an interrupted state, the I/O interface module is connected with the storage module through the dynamic switching unit under the control of the control unit, and the second access signal is transmitted to the storage module through the dynamic switching unit under the control of the control unit. In response to the second access signal, the data generated by the medical device is stored into the storage module through the I/O interface module.

In an embodiment, the control unit is a central processing unit or a microcontroller.

In an embodiment, the processing control module includes a control unit and a dynamic switching unit. The control unit is electrically connected with the wireless transmission module, the dynamic switching unit is electrically connected between the control unit and the storage module, the dynamic switching unit includes a register, and the medical device includes a first access controller and a second access controller. Under control of the control unit, the first access controller or the second access controller is selectively connected with the storage module through the dynamic switching unit. When the second access controller issues a third access signal, the third access signal is temporarily stored in the register. When the dynamic switching unit detects that the first access controller is in an idle state, the second access controller is connected with the storage module through the dynamic switching unit under the control of the control unit, and the third access signal is transmitted to the storage module through the dynamic switching unit under the control of the control unit. In response to the third access signal, the medical device performs a data accessing operation on the storage module through the second access controller.

In an embodiment, when the first access controller issues a fourth access signal, the fourth access signal is temporarily stored in the register. When the dynamic switching unit detects that the second access controller is in an interrupted state, the first access controller is connected with the storage module through the dynamic switching unit under the control of the control unit, and the fourth access signal is transmitted to the storage module through the dynamic switching unit under the control of the control unit. In response to the fourth access signal, the medical device performs a data accessing operation on the storage module through the first access controller.

In an embodiment, the external device includes a man-machine operation interface. The data generated by the medical device is displayed on the man-machine operation interface, or the medical device is controlled according to a control command that is inputted through the man-machine operation interface.

From the above descriptions, the storage device includes the storage module and the wireless transmission module. After the storage device is installed in the medical device, the data generated by the medical device can be directly stored in the storage module. In addition, the data stored in the storage module can be transmitted to the external device through the wireless transmission module. The data can be displayed on the man-machine operation interface of the external device. When the wireless transmission module of the storage device is in communication with the external device, the user can input control commands to control the medical device through the external device. Due to the dynamic switching unit of the storage device, the data can be accessed in a multiplexing manner. Consequently, the convenience of accessing data between the storage device and the medical device is enhanced.

The above objects and advantages of the present invention will become more readily apparent to those ordinarily skilled in the art after reviewing the following detailed description and accompanying drawings, in which:

### BRIEF DESCRIPTION OF THE DRAWINGS

FIG. 1 is a schematic functional block diagram illustrating a storage device in a medical device according to a first embodiment of the present invention;
FIG. 2 is a schematic functional block diagram illustrating a storage device in a medical device according to a second embodiment of the present invention;
FIG. 3 is a schematic functional block diagram illustrating a storage device in a medical device according to a third embodiment of the present invention; and
FIG. 4 is a schematic functional block diagram illustrating a medical instrument according to an embodiment of the present invention.

### DETAILED DESCRIPTION OF THE PREFERRED EMBODIMENT

In the following detailed description of the preferred embodiments, reference is made to the accompanying drawings which form a part hereof, and in which are shown by way of illustration specific embodiments in which the invention may be practiced. In this regard, directional terminology, such as "top," "bottom," "front," "back," etc., is used with reference to the orientation of the figures being described. The components of the present invention may be located in a number of different orientations. As such, the directional terminology is used for purposes of illustration and is in no way limiting.

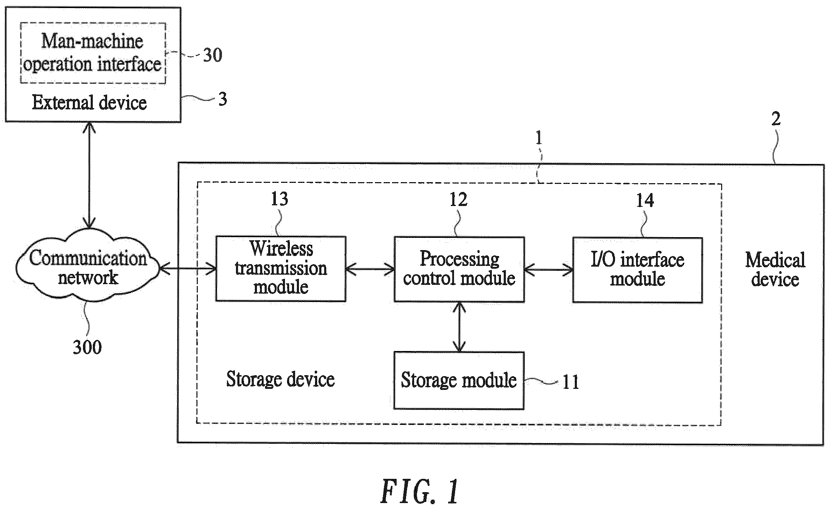

FIG. 1 is a schematic functional block diagram illustrating a storage device in a medical device according to a first embodiment of the present invention. As shown in FIG. 1, the storage device 1 comprises a storage module 11, a processing control module 12 and a wireless transmission module 13. The storage module 11 is used for storing the data that is generated by the medical device 2. The processing control module 12 is electrically connected with the storage module 11. The wireless transmission module 13 is electrically connected with the processing control module 12. Under control of the processing control module 12, the wireless transmission module 13 acquires the data from the storage module 11 and transmits the data to an external device 3. As shown in FIG. 1, the storage device 1 further comprises an I/O interface module 14. The I/O interface module 14 is electrically connected with the medical device 2 and the processing control module 12. Under control of the processing control module 12, the I/O interface module 14 acquires the data from the medical device 2 and stores the data into the storage module 11.

After the processing control module 12 receives a connection request from the user, the processing control module 12 controls the wireless transmission module 13 to connect to the communication network 300. Consequently, the wireless transmission module 13 is connected to the external device 3 through the communication network 300, and the acquired data is transmitted from the wireless transmission module 13 to the external device 3. Preferably but not exclusively, the wireless transmission module 13 is a worldwide interoperability for microwave access (Wimax) module, a Wi-Fi module, a radio frequency (RF) module, a global positioning system (GPS) module or a global system for mobile communications (GSM) module.

In an embodiment, the storage device 1 is a secure digital memory card (also abbreviated as SD card), a Micro SD card, an external hard drive or a flash drive. It is noted that the type and the specification of the storage device 1 are not restricted. Preferably but not exclusively, the processing control module 12 is a central processing unit or a microcontroller.

An example of the medical device 2 includes but is not limited to an ultrasonic scanner, a ventilator, a fundus camera, a continuous positive airway pressure (CPAP) machine or a LED spectrum analyzer. It is noted that the type and the specification of the medical device 2 are not restricted.

In case that the medical device 2 is a ventilator, the generated data of the medical device 2 is the real-time data about the user's respiratory status during the operation of the ventilator. For example, the real-time data includes flowrate, resistance, pressure and any other appropriate operating data. That is, the real-time data include the so-called respiratory parameters. The respiratory parameters contain the oxyhemoglobin saturation by pulse oximetry (SPO2) of the user, the current spontaneous tidal volume value of the user and the current spontaneous breath number of the user. The contents of the respiratory parameters may be determined according to the practical requirements. For example, the respiratory parameters further contain the positive end expiratory pressure (PEEP), the airway resistance (Rexp), the expiratory flow (Fexp) and the expiratory flow waveform information. It is noted that the examples of the respiratory parameters are not restricted.

**As** shown in FIG. 1, the external device 3 comprises a man-machine operation interface 30. When the data stored in the storage module 11 is transmitted to the external device 3 through the wireless transmission module 13 of the storage device 1, the data can be displayed on the man-machine operation interface 30 of the external device 3. In addition, the user can input control commands to control the medical device 2 through the man-machine operation interface 30 of the external device 3. For example, according to the control command, the medical device 2 is powered on or powered off, or the operating parameters of the medical device 2 are set. An example of the external device 3 includes but is not limited to a smart phone, a tablet computer, a notebook computer or a personal computer. Preferably but not exclusively, the man-machine operation interface 30 is a graphic operation interface that is generated after an operation system or an application program loaded to the external device 3 is executed.

As mentioned above, after the storage device 1 is installed in the medical device 2, the data generated by the medical device 2 can be directly stored in the storage module 11 of the storage device 1. In addition, the data stored in the storage module 11 can be transmitted to the external device 3 through the wireless transmission module 13 of the storage device 1. In other words, the use of the storage device 1 increases the storage space of the medical device 2 and allows the medical device 2 to connect to the external device 3 through the communication network 300. Due to this design, the medical practitioners can directly observe the generated data of the medical device 2 through the external device 3 and remotely control various functions of the medical device 2 through the external device 3. Consequently, the work efficiency of medical practitioners will be largely enhanced.

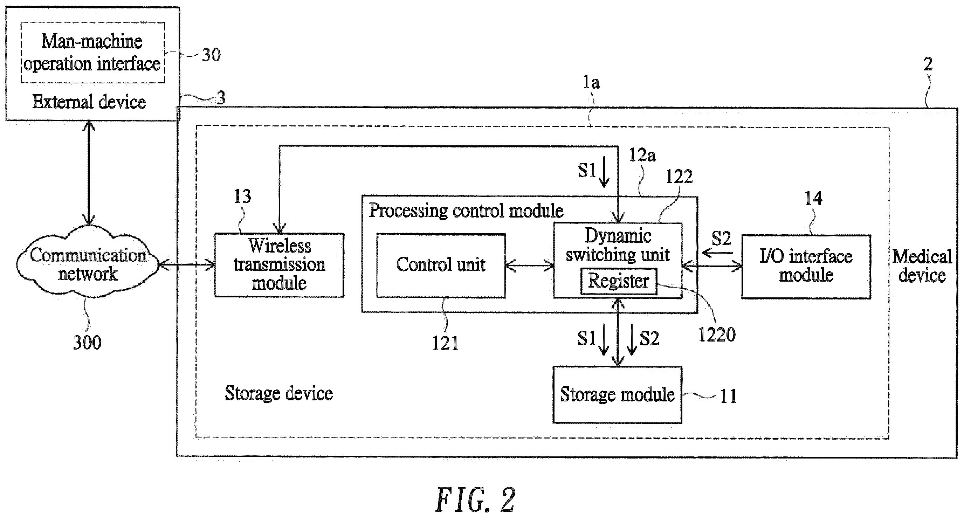

FIG. 2 is a schematic functional block diagram illustrating a storage device in a medical device according to a second embodiment of the present invention. In comparison with the storage device of FIG. 1, the processing control module 12a in the storage device 1a of this embodiment comprises a control unit 121 and a dynamic switching unit 122. The dynamic switching unit 122 is electrically connected between the control unit 121, the storage module 11, the I/O interface module 14 and the wireless transmission module 13. In addition, the dynamic switching unit 122 comprises a register 1220. After the storage device 1a is installed in the medical device 2, the I/O interface module 14 of the storage device 1a is electrically connected with the medical device 2. The data generated by the medical device 2 can be stored into the storage module 11 through the I/O interface 14. In addition, the dynamic switching unit 122 is electrically connected with the I/O interface module 14 to continuously detect the operating status of the I/O interface module 14.

When the user intends to transmit the generated data of the medical device 2 to the external device 3 through the wireless transmission module 13, the wireless transmission module 13 issues a first access signal S1 to the dynamic switching unit 122. The first access signal S1 is temporarily stored in the register 1220. When the dynamic switching unit 122 detects that the operating state of the I/O interface module 14 is an idle state, it means that no data are transmitted between the I/O interface module 14 and the medical device 2. Meanwhile, the wireless transmission module 13 is electrically connected with the storage module 11 through the dynamic switching unit 122 under the control of the control unit 121. In addition, the first access signal S 1 is transmitted from the register 1220 to the storage module 11 through the dynamic switching unit 122 under the control of the control unit 121. In response to the first access signal S1, the data is transmitted from the storage module 11 to the external device 3 through the wireless transmission module 13.

When the wireless transmission module 13 and the storage module 11 are connected with each other through the dynamic switching unit 122, the dynamic switching unit 122 continuously detects the operating state of the wireless transmission module 13. When a new data is generated by the medical device 2 and ready to be stored into the storage module 11, the I/O interface module 14 issues a second access signal S2 to the dynamic switching unit 122. The second access signal S2 is temporarily stored in the register 1220. When the dynamic switching unit 122 detects that the operating state of the wireless transmission module 13 is an interrupted state, it means that no data are transmitted between the wireless transmission module 13 and the external device 3 or the data transmission process is interrupted. Meanwhile, the I/O interface module 14 is electrically connected with the storage module 11 through the dynamic switching unit 122 under the control of the control unit 121. In addition, the second access signal S2 is transmitted from the register 1220 to the storage module 11 through the dynamic switching unit 122 under the control of the control unit 121. In response to the second access signal S2, the data is transmitted from the medical device 2 to the storage module 11 through the I/O interface module 14.

Preferably but not exclusively, the control unit 121 is a central processing unit or a microcontroller.

As mentioned above, after the storage device 1a is installed in the medical device 2, the data generated by the medical device 2 can be directly stored in the storage module 11 of the storage device 1a. In addition, the data stored in the storage module 11 can be transmitted to the external device 3 through the wireless transmission module 13 of the storage device 1a. Moreover, the dynamic switching unit 122 of the processing control module 12a detects the operating state of the I/O interface module 14 and the operating state of the wireless transmission module 13. According to the operating state, the I/O interface module 14 or the wireless transmission module 13 is electrically connected with the storage module 11 through the dynamic switching unit 122 under the control of the control unit 121. In such way, the storage device 1a has the multiplexing function, and the convenience of accessing data is enhanced.

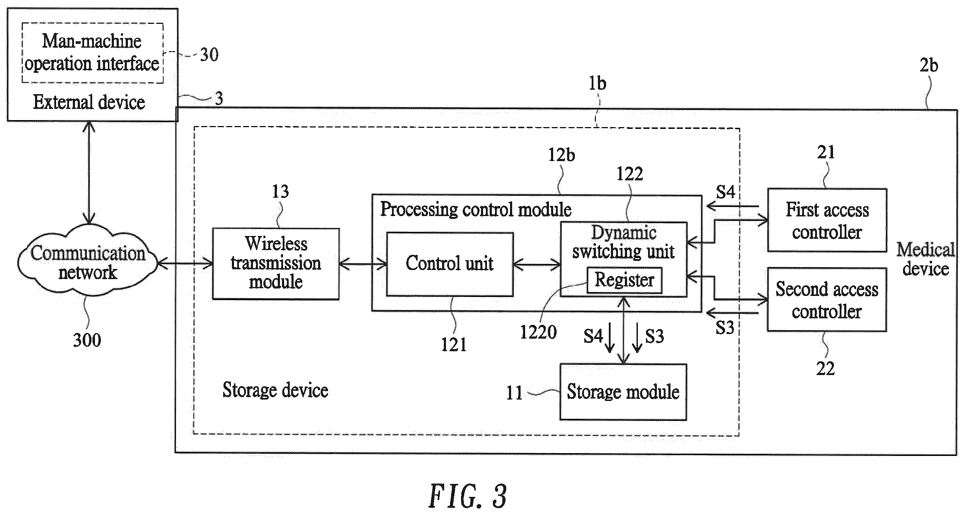

FIG. 3 is a schematic functional block diagram illustrating a storage device in a medical device according to a third embodiment of the present invention. In comparison with the storage device of FIG. 1, the processing control module 12b in the storage device 1b of this embodiment comprises a control unit 121 and a dynamic switching unit 122. In addition, the medical device 2b further comprises a first access controller 21 and a second access controller 22. The dynamic switching unit 122 is electrically connected between the control unit 121 and the storage module 11. In addition, the dynamic switching unit 122 comprises a register 1220. Under control of the control unit 121, the first access controller 21 or the second access controller 22 is selectively connected with the storage module 11 through the dynamic switching unit 122. When the second access controller 22 issues a third access signal S3, the third access signal S3 is temporarily stored in the register 1220. When the dynamic switching unit 122 detects that the operating state of the first access controller 21 is an idle state, the second access controller 22 is connected with the storage module 11 through the dynamic switching unit 122 under the control of the control unit 121. In addition, the third access signal S3 is transmitted from the register 1220 to the storage module 11 through the dynamic switching unit 122 under the control of the control unit 121. In response to the third access signal S3, the medical device 2 performs a data accessing operation on the storage module 11 of the storage device 1b through the second access controller 22. For example, while the data accessing operation is performed, the data is transmitted from the storage module 11 to the external device 3 through the wireless transmission module 13.

When the second access controller 22 is connected with the storage module 11 through the dynamic switching unit 122, the dynamic switching unit 122 continuously detects the operating state of the second access controller 22. When the first access controller 21 issues a fourth access signal S4, the fourth access signal S4 is temporarily stored in the register 1220. When the dynamic switching unit 122 detects that the operating state of the second access controller 22 is an interrupted state, the first access controller 21 is connected with the storage module 11 through the dynamic switching unit 122 under the control of the control unit 121. In addition, the fourth access signal S4 is transmitted from the register 1220 to the storage module 11 through the dynamic switching unit 122 under the control of the control unit 121. In response to the fourth access signal S4, the medical device 2 performs a data accessing operation on the storage module 11 of the storage device 1b through the first access controller 21. For example, while the data accessing operation is performed, the new data generated by the medical device 2b is stored into the storage module 11 through the I/O interface module 14.

As mentioned above, after the storage device 1b is installed in the medical device 2b, the data generated by the medical device 2b can be directly stored in the storage module 11 of the storage device 1b. In addition, the data stored in the storage module 11 can be transmitted to the external device 3 through the wireless transmission module 13 of the storage device 1b. Moreover, the dynamic switching unit 122 of the processing control module 12b detects the operating state of the first access controller 21 and the operating state of the second access controller 22. According to the operating state, the first access controller 21 or the second access controller 22 is selectively connected with the storage module 11 through the dynamic switching unit 122 under the control of the control unit 121. In such way, the storage device 1b and the medical device 2b have the multiplexing functions, and the smoothness of accessing data is enhanced.

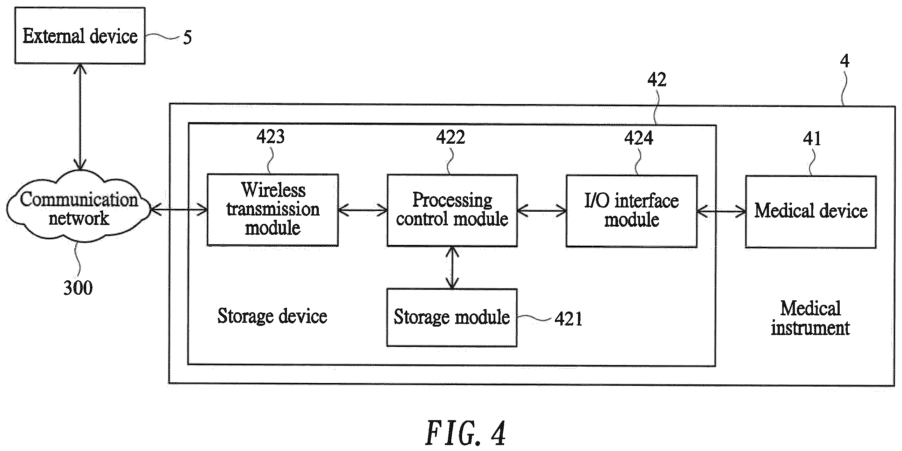

FIG. 4 is a schematic functional block diagram illustrating a medical instrument according to an embodiment of the present invention. As shown in FIG. 4, the medical instrument 4 comprises a medical device 41 and a storage device 42. The storage device 42 is electrically connected with the medical device 41. The storage device 42 comprises a storage module 421, a processing control module 422, a wireless transmission module 423 and an I/O interface module 424. The storage module 421 is used for storing the data that is generated by the medical device 41. The processing control module 422 is electrically connected with the storage module 421. The wireless transmission module 423 is electrically connected with the processing control module 422. Under control of the processing control module 422, the wireless transmission module 423 acquires the data from the storage module 421 and transmits the data to an external device 5. After the processing control module 422 receives a connection request from the user, the processing control module 422 controls the wireless transmission module 423 to connect to the communication network 500. Consequently, the wireless transmission module 423 is connected to the external device 5 through the communication network 500. The I/O interface module 424 is electrically connected with the medical device 41 and the processing control module 422. Under control of the processing control module 422, the I/O interface module 424 acquires the data from the medical device 41 and stores the data into the storage module 421.

In this embodiment, the medical device 41 can be connected with the external device 5 through the storage device 42. In some other embodiments, the storage device 42 has the multiplexing function that is similar to the storage device 1a of FIG. 2 or the storage device 1b of FIG. 3.

From the above descriptions, the storage device includes the storage module and the wireless transmission module. After the storage device is installed in the medical device, the data generated by the medical device can be directly stored in the storage module. In addition, the data stored in the storage module can be transmitted to the external device through the wireless transmission module. The data can be displayed on the man-machine operation interface of the external device. When the wireless transmission module of the storage device is in communication with the external device, the user can input control commands to control the medical device through the external device. Due to the dynamic switching unit of the storage device, the data can be accessed in a multiplexing manner. Consequently, the convenience of accessing data between the storage device and the medical device is enhanced.

While the invention has been described in terms of what is presently considered to be the most practical and preferred embodiments, it is to be understood that the invention needs not be limited to the disclosed embodiments. On the contrary, it is intended to cover various modifications and similar arrangements included within the spirit and scope of the appended claims which are to be accorded with the broadest interpretation so as to encompass all modifications and similar structures.

## Claims

1. A storage device (1, 1a, 1b) installed in a medical device (2, 2b), **characterized by** comprising:
a storage module (11), wherein a data generated by the medical device (2, 2b) is stored in the storage module (11);
a processing control module (12, 12a, 12b) electrically connected with the storage module (11); and
a wireless transmission module (13) electrically connected with the processing control module (12, 12a, 12b), wherein under control of the processing control module (12, 12a, 12b), the data is acquired from the storage module (11) and transmitted to an external device (3) through the wireless transmission module (13).

2. The storage device (1, 1a, 1b) according to claim 1, **characterized in that** the storage device (1, 1a, 1b) further comprises an I/O interface module (14), and the I/O interface module (14) is electrically connected with the medical device (2, 2b) and the processing control module (12, 12a, 12b), wherein under control of the processing control module (12, 12a, 12b), the data from the medical device (2, 2b) is transmitted and stored to the storage module (11) through the I/O interface module (14).

3. The storage device (1, 1a, 1b) according to claim 2, **characterized in that** the processing control module (12, 12a, 12b) comprises a control unit (121) and a dynamic switching unit (122), wherein the dynamic switching unit (122) is electrically connected between the control unit (121), the storage module (11), the I/O interface module (14) and the wireless transmission module (13), and the dynamic switching unit (122) comprises a register (1220), wherein when the wireless transmission module (13) issues a first access signal (S1) to the dynamic switching unit (122), the first access signal (S1) is temporarily stored in the register (1220), wherein when the dynamic switching unit (122) detects that the I/O interface module (14) is in an idle state, the wireless transmission module (13) is connected with the storage module (11) through the dynamic switching unit (122) under the control of the control unit (121), and the first access signal (S1) is transmitted to the storage module (11) through the dynamic switching unit (122) under the control of the control unit (121), wherein in response to the first access signal (S1), the data is acquired from the storage module (11) through the wireless transmission module (13), wherein when the I/O interface module (14) issues a second access signal (S2) to the dynamic switching unit (122), the second access signal (S2) is temporarily stored in the register (1220), wherein when the dynamic switching unit (122) detects that the wireless transmission module (13) is in an interrupted state, the I/O interface module (14) is connected with the storage module (11) through the dynamic switching unit (122) under the control of the control unit (121), and the second access signal (S2) is transmitted to the storage module (11) through the dynamic switching unit (122) under the control of the control unit (121), wherein in response to the second access signal (S2), the data generated by the medical device (2, 2b) is stored into the storage module (11) through the I/O interface module (14).

4. The storage device (1, 1a, 1b) according to claim 3, **characterized in that** the control unit (121) is a central processing unit or a microcontroller.

5. The storage device (1, 1a, 1b) according to claim 1, **characterized in that** the processing control module (12, 12a, 12b) comprises a control unit (121) and a dynamic switching unit (122), wherein the control unit (121) is electrically connected with the wireless transmission module (13), the dynamic switching unit (122) is electrically connected between the control unit (121) and the storage module (11), the dynamic switching unit (122) comprises a register (1220), and the medical device (2, 2b) comprises a first access controller (21) and a second access controller (22), wherein under control of the control unit (121), the first access controller (21) or the second access controller (22) is selectively connected with the storage module (11) through the dynamic switching unit (122), wherein when the second access controller (22) issues a third access signal (S3), the third access signal (S3) is temporarily stored in the register (1220), wherein when the dynamic switching unit (122) detects that the first access controller (21) is in an idle state, the second access controller (22) is connected with the storage module (11) through the dynamic switching unit (122) under the control of the control unit (121), and the third access signal (S3) is transmitted to the storage module (11) through the dynamic switching unit (122) under the control of the control unit (121), wherein in response to the third access signal (S3), the medical device (2, 2b) performs a data accessing operation on the storage module (11) through the second access controller (22), wherein when the first access controller (21) issues a fourth access signal (S4), the fourth access signal (S4) is temporarily stored in the register (1220), wherein when the dynamic switching unit (122) detects that the second access controller (22) is in an interrupted state, the first access controller (21) is connected with the storage module (11) through the dynamic switching unit (122) under the control of the control unit (121), and the fourth access signal (S4) is transmitted to the storage module (11) through the dynamic switching unit (122) under the control of the control unit (121), where in response to the fourth access signal (S4), the medical device (2, 2b) performs a data accessing operation on the storage module (11) through the first access controller (21).

6. The storage device (1, 1a, 1b) according to claim 1, **characterized in that** the external device (3) comprises a man-machine operation interface (300), wherein the data generated by the medical device (2, 2b) is displayed on the man-machine operation interface (300), or the medical device (2, 2b) is controlled according to a control command that is inputted through the man-machine operation interface (300).

7. The storage device (1, 1a, 1b) according to claim 1, **characterized in that** the processing control module (12, 12a, 12b) is a central processing unit or a microcontroller.

8. The storage device (1, 1a, 1b) according to claim 1, **characterized in that** the external device (3) is a smart phone, a tablet computer, a notebook computer or a personal computer.

9. The storage device (1, 1a, 1b) according to claim 1, **characterized in that** the wireless transmission module (13) is a worldwide interoperability for microwave access (Wimax) module, a Wi-Fi module, a radio frequency (RF) module, a global positioning system (GPS) module or a global system for mobile communications (GSM) module.

10. The storage device (1, 1a, 1b) according to claim 1, **characterized in that** the medical device (2, 2b) is an ultrasonic scanner, a ventilator, a fundus camera, a continuous positive airway pressure machine or a LED spectrum analyzer.

11. A medical instrument (4), **characterized by** comprising:
a medical device (41); and
a storage device (42) electrically connected with the medical device (41), and comprising a storage module (421), a processing control module (422) and a wireless transmission module (43), wherein a data generated by the medical device (41) is stored in the storage module (421), the processing control module (422) is electrically connected with the storage module (421), and the wireless transmission module (43) is electrically connected with the processing control module (422), wherein under control of the processing control module (422), the data is acquired from the storage module (421) and transmitted to an external device (5) through the wireless transmission module (43).

12. The medical instrument (4) according to claim 11, **characterized in that** the storage device (42) further comprises an I/O interface module (424), and the I/O interface module (424) is electrically connected with the medical device (41) and the processing control module (422), wherein under control of the processing control module (422), the data from the medical device (41) is transmitted and stored to the storage module (421) through the I/O interface module (424).

13. The medical instrument (4) according to claim 12, **characterized in that** the processing control module (422) comprises a control unit (121) and a dynamic switching unit (122), wherein the dynamic switching unit (122) is electrically connected between the control unit (121), the storage module (421), the I/O interface module (424) and the wireless transmission module (43), and the dynamic switching unit (122) comprises a register (1220), wherein when the wireless transmission module (43) issues a first access signal (S1) to the dynamic switching unit (122), the first access signal (S1) is temporarily stored in the register (1220), wherein when the dynamic switching unit (122) detects that the I/O interface module (424) is in an idle state, the wireless transmission module (43) is connected with the storage module (421) through the dynamic switching unit (122) under the control of the control unit (121), and the first access signal (S1) is transmitted to the storage module (421) through the dynamic switching unit (122) under the control of the control unit (121), wherein in response to the first access signal (S1), the data is acquired from the storage module (421) through the wireless transmission module (43), wherein when the I/O interface module (424) issues a second access signal (S2) to the dynamic switching unit (122), the second access signal (S2) is temporarily stored in the register (1220), wherein when the dynamic switching unit (122) detects that the wireless transmission module (43) is in an interrupted state, the I/O interface module (424) is connected with the storage module (421) through the dynamic switching unit (122) under the control of the control unit (121), and the second access signal (S2) is transmitted to the storage module (421) through the dynamic switching unit (122) under the control of the control unit (121), wherein in response to the second access signal (S2), the data generated by the medical device (41) is stored into the storage module (421) through the I/O interface module (424).

14. The medical instrument (4) according to claim 13, **characterized in that** the control unit (121) is a central processing unit or a microcontroller.

15. The medical instrument (4) according to claim 11, **characterized in that** the processing control module (422) comprises a control unit (121) and a dynamic switching unit (122), wherein the control unit (121) is electrically connected with the wireless transmission module (43), the dynamic switching unit (122) is electrically connected between the control unit (121) and the storage module (421), the dynamic switching unit (122) comprises a register (1220), and the medical device (41) comprises a first access controller (21) and a second access controller (22), wherein under control of the control unit (121), the first access controller (21) or the second access controller (22) is selectively connected with the storage module (421) through the dynamic switching unit (122), wherein when the second access controller (22) issues a third access signal (S3), the third access signal (S3) is temporarily stored in the register (1220), wherein when the dynamic switching unit (122) detects that the first access controller (21) is in an idle state, the second access controller (22) is connected with the storage module (421) through the dynamic switching unit (122) under the control of the control unit (121), and the third access signal (S3) is transmitted to the storage module (421) through the dynamic switching unit (122) under the control of the control unit (121), wherein in response to the third access signal (S3), the medical device (41) performs a data accessing operation on the storage module (421) through the second access controller (22), wherein when the first access controller (21) issues a fourth access signal (S4), the fourth access signal (S4) is temporarily stored in the register (1220), wherein when the dynamic switching unit (122) detects that the second access controller (22) is in an interrupted state, the first access controller (21) is connected with the storage module (421) through the dynamic switching unit (122) under the control of the control unit (121), and the fourth access signal (S4) is transmitted to the storage module (421) through the dynamic switching unit (122) under the control of the control unit (121), where in response to the fourth access signal (S4), the medical device (41) performs a data accessing operation on the storage module (421) through the first access controller (21).
